(19) Europäisches Patentamt
European Patent Office
Office européen des brevets

(11) Veröffentlichungsnummer : **0 150 157**
**B1**

(12) # EUROPÄISCHE PATENTSCHRIFT

(45) Veröffentlichungstag der Patentschrift : **27.07.88**

(51) Int. Cl.⁴ : **C 07 J 53/00**, A 61 K 31/585

(21) Anmeldenummer : **85730004.0**

(22) Anmeldetag : **17.01.85**

(54) **6,6-Ethylen-15,16-methylen-3-oxo-17alpha-pregn-4-en-21,17-carbolactone, Verfahren zu deren Herstellung und diese enthaltende pharmazeutische Präparate.**

(30) Priorität : **20.01.84 DE 3402329**

(43) Veröffentlichungstag der Anmeldung : **31.07.85 Patentblatt 85/31**

(45) Bekanntmachung des Hinweises auf die Patenterteilung : **27.07.88 Patentblatt 88/30**

(84) Benannte Vertragsstaaten : **AT BE CH DE FR GB IT LI LU NL SE**

(56) Entgegenhaltungen :
**FR-A- 2 370 755**
**US-A- 3 422 097**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Nickisch, Klaus, Dr.**
**Alt Lichtenrade 11**
**D-1000 Berlin 49 (DE)**
Erfinder : **Bittler, Dieter**
**Bölkauer Pfad 11**
**D-1000 Berlin 27 (DE)**
Erfinder : **Laurent, Henry, Dr.**
**Glambecker Weg 21**
**D-1000 Berlin 28 (DE)**
Erfinder : **Wiechert, Rudolf, Prof.-Dr.**
**Petzower Strasse 8 A**
**D-1000 Berlin 39 (DE)**
Erfinder : **Beier, Sybille, Dr.**
**Uhlandstrasse 121**
**D-1000 Berlin 31 (DE)**
Erfinder : **Elger, Walter, Dr.**
**Schorlemer Allee 12 B**
**D-1000 Berlin 33 (DE)**

**EP 0 150 157 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 6,6-Ethylen-15,16-methylen-3-oxo-17α-pregn-4-en-21,17-carbolactone der allgemeinen Formel I, Verfahren zu deren Herstellung und diese enthaltende pharmazeutische Präparate gemäß der Patentansprüche.

Die neuen 6,6-Ethylen-15,16-methylen-3-oxo-17α-pregn-4-en-21,17-carbolactone werden durch die allgemeine Formel I gekennzeichnet

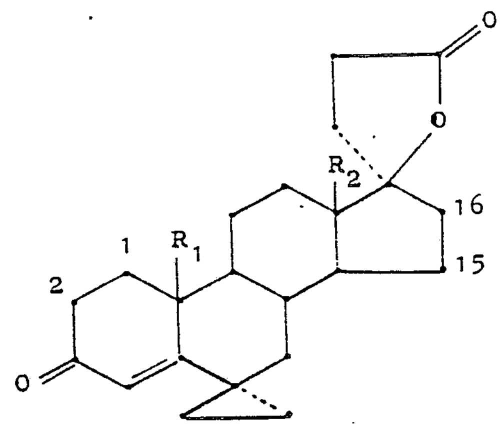

(I)

worin

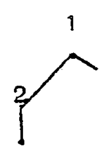

eine CC-Einfach- oder CC-Doppelbindung,

$R_1$ ein Wasserstoffatom oder eine Methylgruppe,

$R_2$ eine Methyl- oder Ethylgruppe und

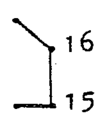 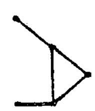 oder 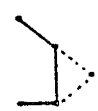

bedeuten.

Die neuen Verbindungen der allgemeinen Formel I weisen neben einer hohen aldosteronantagonistischen Wirkung eine starke gestagene Potenz auf. Die Kombination von aldosteronantagonistischer und gestagener Wirkung wird bei keinem der bekannten synthetischen Gestagene der Ethinyl-nortesto- bzw. Hydroxyprogesteron-Reihe, wohl aber bei dem natürlichen Gestagen, dem Progesteron gefunden. Da die neuen Verbindungen das progesteronartige Wirkungsprofil besitzen, werden bei ihrer Verwendung als Kontrazeptiva die sonst auftretenden Nebenwirkungen, wie Blutdruckanstieg und Ödeme, nicht in Erscheinung treten.

Aus der U.S.-Patentschrift 3 422 097 sind 6,6-Ethylen-21,17-carbolactone bekannt, die in 15,16-Stellung keine Methylengruppe enthalten. Von den Verbindungen wird berichtet, daß sie androgen und anabol wirksam sind und Natrium-uretische Aktivität besitzen.

Verbindungen, die statt der 6,6-Ethylengruppe eine 6β,7β-Methylengruppe enthalten, werden in der publizierten französischen Patentanmeldung FR-A-2 370 755 und der deutschen Offenlegungsschrift DE-A-3 022 337 beschrieben. Die Verbindungen (siehe die Vergleichssubstanz in der Tabelle auf Seite 19) zeigen ebenfalls aldosteronantagonistische und gestagene Wirkung, wobei die gestagene Wirkung aber nicht so stark ausgeprägt ist wie bei den erfindungsgemäßen Verbindungen.

Im Test auf Antialdosteronwirkung erweisen sich die neuen Verbindungen der allgemeinen Formel I bis zu 5 mal stärker wirksam als Spironolacton.

Im modifizierten Clauberg-Test auf gestagene Wirkung werden nach subkutaner Applikation der erfindungsgemäßen Verbindungen positive Ergebnisse mit Mengen von 0,03 mg erzielt.

Im Schwangerschaftserhaltungstest an Ratten und Mäusen liegt die zur Erzielung eines positiven Effekts benötigte Mindestmenge bei 0,1 mg.

Die neuen Verbindungen der allgemeinen Formel I können allein oder in Kombination mit Östrogenen

in Präparaten zur Kontrazeption verwendet werden. Erfindungsgemäß sollen die neuen Verbindungen besonders bei Frauen eingesetzt werden, die eine Kontrazeption wünschen und bei denen ein Blutdruckanstieg aufgrund vorhandener Risikofaktoren, wie fortgeschrittenes Alter, Übergewicht oder Rauchen, wahrscheinlich ist. Aufgrund des dem natürlichen Progesteron vergleichbaren Profils dieser Substanzen sollte auch bei Frauen, die nicht als Risikopatientinnen eingestuft werden können, das subjektive Wohlbefinden und die Verträglichkeit gegenüber bekannten Präparaten erhöht werden.

Die Dosierung der erfindungsgemäßen Verbindungen in Kontrazeptionspräparaten soll vorzugsweise 0,5 bis 5 mg pro Tag betragen.

Die gestagenen und östrogenen Wirkstoffkomponenten werden in Kontrazeptionspräparaten vorzugsweise zusammen oral appliziert. Die tägliche Dosis wird vorzugsweise einmalig verabfolgt.

Das Östrogen wird in einer Menge verabfolgt, die der von 0,03 bis 0,05 mg Ethinylöstradiol entspricht.

Die neuen Verbindungen der allgemeinen Formel I können auch in Präparaten zur Behandlung gynäkologischer Störungen eingesetzt werden. Wegen ihres günstigen Wirkungsprofils sind die erfindungsgemäßen Verbindungen besonders gut geeignet zur Behandlung praemenstrueller Beschwerden, wie Kopfschmerzen, depressive Verstimmungen, Wasserretention und Mastodynie. Die Tagesdosis bei der Behandlung praemenstrueller Beschwerden liegt bei etwa 1 bis 20 mg.

Die Formulierung der pharmazeutischen Präparate auf Basis der neuen Verbindungen erfolgt in an sich bekannter Weise, indem man den Wirkstoff, gegebenenfalls in Kombination mit einem Östrogen, mit den in der Galenik gebräuchlichen Trägersubstanzen, Verdünnungsmitteln, gegebenenfalls Geschmackskorrigentien usw. verarbeitet und in die gewünschte Applikationsform überführt.

Für die bevorzugte orale Applikation kommen insbesondere Tabletten, Dragées, Kapseln, Pillen, Suspensionen oder Lösungen infrage.

Für die parenterale Applikation sind insbesondere ölige Lösungen, wie zum Beispiel Lösungen in Sesamöl, Rizinusöl und Baumwollsamenöl geeignet. Zur Erhöhung der Löslichkeit können Lösungsvermittler, wie zum Beispiel Benzylbenzoat oder Benzylalkohol, zugesetzt werden.

Die erfindungsgemäßen Verbindungen der allgemeinen Formel I werden dadurch hergestellt, daß man in 15,16-Methylen-3-oxo-17α-pregn-4-en-21,17-carbolactone der allgemeinen Formel II

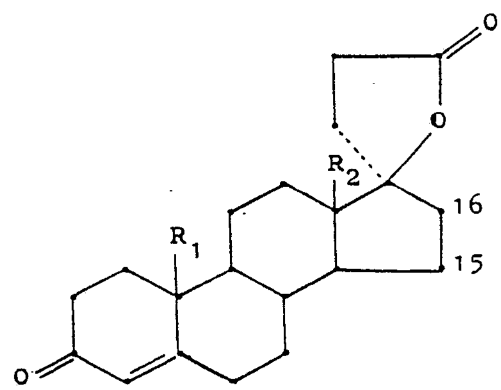

(II)

worin

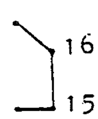

$R_1$ und $R_2$ die in Formel I angegebene Bedeutung haben, über die 3,5-Dien-3-Amine mit Formalin unter Rückbildung des 4-En-3-Oxo-Systems eine 6α-Hydroxymethylgruppe einführt, aus der 6α-Hydroxymethylverbindung die 6-Methylenverbindung bildet und diese zur 6,6-Ethylenverbindung methyleniert und gegebenenfalls die $\Delta^1$-Doppelbindung einführt.

Zunächst wird das $\Delta^4$-3-Keton mit einer sekundären Base in das entsprechende $\Delta^{3,5}$-3-Amin überführt.

Als sekundäre Basen sind zum Beispiel Diethylamin, Anilin, Pyrrolidin und Morpholin geeignet.

Zur Einführung der 6α-Hydroxymethylgruppe wird das $\Delta^{3,5}$-3-Amin in alkoholischer Lösung mit Formalin behandelt (vgl. Helv. Chim. Acta 56 (1973) 2396).

Aus der 6α-Hydroxymethylverbindung wird mit Salzsäure in Dioxan Wasser abgespalten zur entsprechenden 6-Methylenverbindung. Die Wasserabspaltung kann auch in der Weise vorgenommen werden, daß zunächst eine Fluchtgruppe eingeführt und wieder abgespalten wird.

Als Fluchtgruppen sind zum Beispiel Mesylat, Tosylat und Benzoat geeignet.

Die Methylenierung der 6-Methylen- zur 6,6-Ethylenverbindung erfolgt mit Dimethylsulfoxoniummethylid. Hierzu wird das 6-Methylen-Steroid zu einer Suspension von Trimethylsulfoxoniumjodid mit Natriumhydrid in Mineralöl und Dimethylsulfoxid oder zu einer Lösung von Trimethylsulfoxoniumjodid

und Natriumhydroxid in Dimethylsulfoxid gegeben. Die Reaktion ist nach 15 bis 60 Minuten bei 20-40 °C beendet (vgl. J. Am. Chem. Soc. 84 (1962) 866-868).

Die sich gegebenenfalls anschließende Einführung der $\Delta^1$-Doppelbindung erfolgt nach an sich bekannten Methoden und kann auf chemischem oder mikrobiologischem Wege erfolgen.

Geeignete chemische Dehydrierungsmittel sind beispielsweise Selendioxid, 2,3-Dichlor-5,6-dicyanobenzochinon, Chloranil, Thalliumtriacetat oder Bleitetraacetat.

Geeignete Mikroorganismen für die 1,2-Dehydrierung sind beispielsweise Schizomyceten, insbesondere solche der Genera Arthrobacter, wie zum Beispiel A. simplex (ATCC 6946) ; Bacillus, wie zum Beispiel B. lentus (ATCC 13805) und B. sphaericus (ATCC 7055) ; Pseudomonas, wie zum Beispiel P. aeruginosa (IFO 3505) ; Flavobacterium, wie zum Beispiel F. flavescens (IFO 3058) ; Lactobacillus, wie zum Beispiel L. brevis (IFO 3345) und Nocardia, wie zum Beispiel N. opaca (ATCC 4276).

Die 1,2-Dehydrierung wird bevorzugt chemisch ausgeführt. Hierzu wird das 1,2-Dihydrosteroid in einem geeigneten Lösungsmittel mit dem Dehydrierungsmittel über längere Zeit erhitzt. Geeignete Lösungsmittel sind beispielsweise Dioxan, tert. Butanol, Tetrahydrofuran, Toluol, Benzol bzw. Gemische dieser Lösungsmittel.

Die Reaktion ist nach mehreren Stunden beendet. Es empfiehlt sich, die Umsetzung durch Dünnschichtchromatographie zu verfolgen. Das Reaktionsgemisch wird aufgearbeitet, wenn das Ausgangsmaterial umgesetzt ist.

## Beispiel 1

a) Eine Lösung von 4,1 g, 15β, 16β-Methylen-3-oxo-17α-pregn-4-en-21,17-carbolacton in 31 ml Methanol wird mit 2,05 ml Pyrrolidin 15 Minuten unter Rückfluß erhitzt. Nach dem Abkühlen im Eisbad wird der ausgefallene Niederschlag abgesaugt, mit wenig Methanol nachgewaschen und getrocknet. Es werden 4,5 g 15β, 16β-Methylen-3-pyrrolidino-17α-pregna-3,5-dien-21,17-carbolacton vom Schmelzpunkt 234-237 °C (unter Zersetzung) erhalten.

$$UV : \varepsilon_{276} = 22.900$$

b) Zu einer Lösung von 4,5 g 15β, 16β-Methylen-3-pyrrolidino-17α-pregna-3,5-dien-21,17-carbolacton in 90 ml Ethanol und 45 ml Benzol werden in 5 Minuten bei Raumtemperatur 4,5 ml 37 %ige Formalinlösung zugetropft. Nach einer Reaktionszeit von 30 Minuten wird die Reaktionslösung im Vakuum zur Trockne eingedampft. Der Rückstand wird an Kieselgel chromatographiert, und es werden 3,15 g 6α-Hydroxymethyl-15β, 16β-methylen-3-oxo-17α-pregn-4-en-21,17-carbolacton als Öl erhalten.

$$UV : \varepsilon_{241} = 14.500$$

c) 3,15 g 6α-Hydroxymethyl-15β, 16β-methylen-3-oxo-17α-pregn-4-en-21,17-carbolacton werden in 77 ml Dioxan mit 9,1 ml 5 N Salzsäure 2 Stunden bei Raumtemperatur gerührt. Die Reaktionslösung wird dann mit einem Überschuß an Natriumhydrogencarbonat versetzt, von den ausgefallenen anorganischen Salzen abfiltriert, das Filtrat mit Ether verdünnt und mit Wasser gewaschen. Nach dem Trocknen und Eindampfen wird der Rückstand an Kieselgel chromatographiert. Nach Umkristallisieren aus Diisopropylether/Aceton werden 1,2 g 6 ; 15β, 16β-Dimethylen-3-oxo-17α-pregn-4-en-21,17-carbolacton vom Schmelzpunkt 173,1 °C erhalten.

$$UV : \varepsilon_{261} = 11.300$$

d) 1,41 g Trimethylsulfoxoniumiodid werden mit 239 mg Natriumhydrid (55 %ige Ölsuspension) in 21,5 ml Dimethylsulfoxid (DMSO) 1,5 Stunden bei Raumtemperatur gerührt. Zu dieser Lösung werden unter Argon 1,14 g 6 ; 15β, 16β-Dimethylen-3-oxo-17α-pregn-4-en-21,17-carbolacton gegeben, und es wird 50 Minuten bei Raumtemperatur nachgerührt. Die Reaktionslösung wird dann in Eiswasser eingerührt, mit 2 N Schwefelsäure schwach angesäuert, der ausgefallene Niederschlag abfiltriert und in Methylenchlorid aufgenommen. Nach dem Trocknen und Eindampfen wird der Rückstand an Kieselgel chromatographiert. Nach Umkristallisieren aus Diisopropylether/Aceton werden 725 mg 6,6-Ethylen-15β, 16β-methylen-3-oxo-17α-pregn-4-en-21,17-carbolacton vom Schmelzpunkt 184,2 °C erhalten.

$$UV : \varepsilon_{248} = 14.150$$

## Beispiel 2

Eine Lösung von 670 mg 6,6-Ethylen-15β, 16β-methylen-3-oxo-17α-pregn-4-en-21,17-carbolacton und 670 mg 2,3-Dichlor-5,6-dicyan-p-benzochinon in 13,4 ml Toluol wird 5 Stunden bei 100 °C gerührt. Die Reaktionslösung wird dann mit Ether verdünnt, mit Wasser, Natriumhydrogencarbonatlösung und Wasser gewaschen, getrocknet und eingedampft. Der Rückstand wird dann an Kieselgel chromatographiert. Nach Umkristallisieren aus Diisopropylether/Aceton werden 445 mg 6,6-Ethylen-15β, 16β-methylen-3-oxo-17α-pregna-1,4-dien-21,17-carbolacton vom Schmelzpunkt 199,7 °C erhalten.

UV : $\varepsilon_{243} = 15.150$

Beispiel 3

a) 5,9 g 15α, 16α-Methylen-3-oxo-17α-pregn-4-en-21,17-carbolacton werden in 40 ml Methanol mit 2,95 ml Pyrrolidin, wie im Beispiel 1 beschrieben, umgesetzt und aufgearbeitet. Es werden 5,9 g 15α, 16α-Methylen-3-pyrrolidino-17α-pregna-3,5-dien-21,17-carbolacton vom Schmelzpunkt 235-237,5 °C (unter Zersetzung) erhalten.

UV : $\varepsilon_{276} = 23.300$

b) 5,9 g 15α, 16α-Methylen-3-pyrrolidino-17α-pregna-3,5-dien-21,17-carbolacton werden in 118 ml Ethanol und 59 ml Benzol mit 5,9 ml 37 %iger Formalinlösung, wie im Beispiel 1 beschrieben, umgesetzt und aufgearbeitet. Es werden 3,7 g 6α-Hydroxymethyl-15α, 16α-methylen-3-oxo-17α-pregn-4-en-21,17-carbolacton erhalten.

c) 3,7 g 6α-Hydroxymethyl-15α, 16α-methylen-3-oxo-17α-pregn-4-en-21,17-carbolacton werden in 90,5 ml Dioxan mit 10,7 ml 5 N Salzsäure 3,5 Stunden bei Raumtemperatur gerührt. Es wird dann, wie im Beispiel 1 beschrieben, aufgearbeitet und gereinigt. Nach Umkristallisieren aus Diisopropylether/Aceton werden 2,26 g 6 ; 15α, 16α-Dimethylen-3-oxo-17α-pregn-4-en-21,17-carbolacton vom Schmelzpunkt 162,5-163,5 °C erhalten.

UV : $\varepsilon_{262} = 11.300$

d) 1,87 g 6 ; 15α, 16α-Dimethylen-3-oxo-17α-pregn-4-en-21, 17-carbolacton werden in 35 ml DMSO mit 2,31 g Trimethylsulfoxoniumiodid und 393 mg Natriumhydrid (55 %ige Ölsuspension), wie im Beispiel 1 beschrieben, umgesetzt und aufgearbeitet. Nach Umkristallisieren aus Diisopropylether/Aceton werden 1,1 g 6,6-Ethylen-15α, 16α-methylen-3-oxo-17α-pregn-4-en-21,17-carbolacton vom Schmelzpunkt 176,6 °C erhalten.

UV : $\varepsilon_{247} = 13.900$

Beispiel 4

600 mg 6,6-Ethylen-15α,16α-methylen-3-oxo-17α-pregn-4-en-21,17-carbolacton werden in 12 ml Toluol mit 600 mg 2,3-Dichlor-5,6-dicyan-p-benzochinon 17 Stunden bei 80 °C gerührt. Es wird dann, wie im Beispiel 3 beschrieben, aufgearbeitet und gereinigt. Es werden 520 mg 6,6-Ethylen-15α, 16α-methylen-3-oxo-17α-pregna-1,4-dien-21,17-carbolacton als Öl erhalten.

UV : $\varepsilon_{242} = 14.600$

Beispiel 5

a) 5,0 g 18-Methyl-15β, 16β-methylen-3-oxo-19-nor-17α-pregn-4-en-21,17-carbolacton werden in 125 ml Methanol mit 2,5 ml Pyrrolidin, wie im Beispiel 1 beschrieben, umgesetzt und aufgearbeitet. Es werden 5,0 g 18-Methyl-15β, 16β-methylen-3-pyrrolidino-19-nor-17α-pregna-3,5-dien-21,17-carbolacton erhalten.

b) 5,0 g 18-Methyl-15β, 16β-methylen-3-pyrrolidino-19-nor-17α-pregna-3,5-dien-21,17-carbolacton werden in 100 ml Ethanol und 50 ml Benzol mit 5 ml 37 %iger Formalinlösung, wie im Beispiel 1 beschrieben, umgesetzt und aufgearbeitet. Es werden 2,33 g 6α-Hydroxymethyl-18-methyl-15β, 16β-methylen-19-nor-17α-pregn-4-en-21,17-carbolacton als Öl erhalten.

UV : $\varepsilon_{239} = 13.200$

c) 372 mg 6α-Hydroxymethyl-18-methyl-15β, 16β-methylen-19-nor-17α-pregn-4-en-21,17-carbolacton werden in 3,7 ml Pyridin mit 572 mg p-Toluolsulfonsäurechlorid 17 Stunden bei Raumtemperatur gerührt. Es werden dann 0,09 ml Wasser zugesetzt, 1 Stunde bei Raumtemperatur nachgerührt und die Reaktionslösung in Eiswasser eingerührt. Der ausgefallene Niederschlag wird abfiltriert, mit Wasser gewaschen und in Methylenchlorid aufgenommen. Nach dem Trocknen und Eindampfen werden 465 mg 18-Methyl-15β, 16β-methylen-6α-tosyloxymethyl-19-nor-17α-pregn-4-en-21,17-carbolacton als Öl erhalten.

UV : $\varepsilon_{226} = 20.600$

d) Zu einer Lösung von 426 mg Trimethylsulfoxoniumiodid in 15 ml DMSO werden 72 mg Natriumhydrid (55 %ige Ölsuspension) gegeben und 1 Stunde bei Raumtemperatur gerührt. In diese Lösung werden dann unter Argon 300 mg 18-Methyl-15β, 16β-methylen-6α-tosyloxymethyl-19-nor-17α-pregn-4-en-21,17-carbolacton gegeben, 15 Minuten nachgerührt und in Eiswasser gefällt. Der Niederschlag wird abfiltriert, in Ether aufgenommen, mit Wasser gewaschen, getrocknet und eingedampft. Nach präparativer Dünnschichtchromatographie werden 145 mg 6,6-Ethylen-18-methyl-15β, 16β-methylen-3-oxo-19-nor-17α-pregn-4-en-21,17-carbolacton vom Schmelzpunkt 165,4 °C erhalten.

UV : $\varepsilon_{247}$ = 14.650

Die Ausgangsverbindung gemäß 5 a) wird wie folgt hergestellt :

Eine Lösung von 102 g 15α-Hydroxy-18-methyl-4-östren-3,17-dion in 500 ml Pyridin wird unter Eiskühlung mit 50,9 ml Benzoylchlorid versetzt und 1 Stunde bei Kühlung nachgerührt. Dann werden 9 ml Wasser zugetropft und eine weitere Stunde nachgerührt. Die Reaktionslösung wird dann in Eiswasser eingerührt, der ausgefallene Niederschlag abgesaugt, mit Wasser gewaschen und getrocknet. Es werden 146 g rohes 15α-Benzoyloxy-18-methyl-4-östren-3,17-dion erhalten.

Eine Lösung von 146 g 15α-Benzoyloxy-18-methyl-4-östren-3,17-dion in 1,46 l Dichlormethan wird mit 441 ml Ethylenglykol, 294 ml o-Ameisensäuretriethylester und 7,3 g p-Toluolsulfonsäure versetzt und 1 Stunde bei 50 °C gerührt. Es werden dann 20 ml Pyridin zugesetzt, es wird mit Ether verdünnt und mit Wasser gewaschen. Nach dem Trocknen und Eindampfen werden 165 g rohes 15α-Benzoyloxy-3,3-ethylendioxy-18-methyl-5 bzw. 5(10)-östren-17-on erhalten.

Zu einer Lösung von 320 g Trimethylsulfoxoniumiodid in 1,5 l DMSO werden 56,3 g pulverisiertes Natriumhydroxid gegeben, und es wird 2,5 Stunden bei Raumtemperatur gerührt. Unter Wasserkühlung werden dann 165 g 15α-Benzoyloxy-3,3-ethylendioxy-18-methyl-5 bzw. 5(10)-östren-17-on in 300 ml DMSO zugegeben, und es wird 45 Minuten nachgerührt. Die Reaktionslösung wird dann in Eiswasser eingerührt, der ausgefallene Niederschlag abfiltriert, in Ether aufgenommen, mit Wasser gewaschen und getrocknet. Der nach dem Eindampfen erhaltene Rückstand wird an Kieselgel chromatographiert. Es werden 79,2 g 3,3-Ethylendioxy-18-methyl-15β, 16β-methylen-5 bzw. 5(10)-östren-17-on als Öl erhalten.

Eine Lösung von 62 g 3,3-Ethylendioxy-18-methyl-15β, 16β-methylen-5 bzw. 5(10)-östren-17-on in 600 ml absoluten Tetrahydrofuran (THF) wird auf -10 °C gekühlt, dann werden unter Argon 186 g Kaliumethylat zugesetzt und anschließend 124 ml destillierter Propargylalkohol zugetropft. Die Reaktionslösung wird 45 Stunden unter Kühlung nachgerührt, dann in Eiswasser eingerührt, mit verdünnter Schwefelsäure angesäuert und mit Dichlormethan extrahiert. Nach dem Trocknen und Eindampfen wird der Rückstand an Kieselgel chromatographiert. Es werden 23 g 3,3-Ethylendioxy-17α-(3-hydroxy-1-propinyl)-18-methyl-15β, 16β-methylen-5 bzw. 5(10)-östren-17β-ol als Öl erhalten.

23 g 3,3-Ethylendioxy-17α-(3-hydroxy-1-propinyl)-18-methyl-15β, 16β-methylen-5 bzw. 5(10)-östren-17β-ol werden in 150 ml 2-Propanol und 150 ml THF gelöst und in Gegenwart von 23 g Raney-Nickel-Katalysator mit Wasserstoff hydriert. Es wird dann vom Katalysator abfiltriert und das Filtrat zur Trockne eingedampft. Als Rückstand werden 23 g rohes 3,3-Ethylendioxy-17α-(3-hydroxypropyl)-18-methyl-15β, 16β-methylen-5 bzw. 5(10)-östren-17β-ol als Öl erhalten.

23 g 3,3-Ethylendioxy-17α-(3-hydroxypropyl)-18-methyl-15β, 16β-methylen-5 bzw. 5(10)-östren-17β-ol werden in 230 ml Dimethylformamid (DMF) mit 69 g Pyridiniumdichromat 24 Stunden bei Raumtemperatur gerührt. Die Reaktionslösung wird dann in Essigester eingerührt, von den ausgefallenen Chromsalzen wird abgesaugt und das Filtrat mit Wasser gewaschen. Nach dem Eindampfen wird der Rückstand an Kieselgel chromatographiert. Es werden 15 g 3,3-Ethylendioxy-18-methyl-15β, 16β-methylen-19-nor-17α-pregn-5 bzw. 5(10)-en-21,17-carbolacton als Öl erhalten.

15 g 3,3-Ethylendioxy-18-methyl-15β, 16β-methylen-19-nor-17α-pregn-5 bzw. 5(10)-en-21,17-carbolacton werden in 150 ml Methanol mit 15 ml 8 Vol. %iger Schwefelsäure 6 Stunden bei Raumtemperatur gerührt. Die Reaktionslösung wird dann mit Ether verdünnt, mit Wasser gewaschen, getrocknet und eingedampft. Der Rückstand wird an Kieselgel chromatographiert und nach Umkristallisieren aus Diisopropylether/Aceton werden 8,5 g 18-Methyl-15β, 16β-methylen-3-oxo-19-nor-17α-pregn-4-en-21,17-carbolacton vom Schmelzpunkt 202,5-205,5 °C erhalten.

UV : $\varepsilon_{240}$ = 17.400

## Beispiel 6

a) Zu einer Lösung von 4,0 g 15β, 16β-Methylen-19-nor-3-oxo-17α-pregn-4-en-21,17-carbolacton in 35 ml siedendem Methanol gibt man 2 ml Pyrrolidin und erhitzt weitere 20 Minuten unter Rückfluß. Nach dem Abkühlen wird der ausgefallene Niederschlag abgesaugt und mit wenig kaltem Methanol nachgewaschen und im Vakuum getrocknet. Man erhält 4,3 g 15β, 16β-Methylen-19-nor-3-pyrrolidino-17α-pregna-3,5-dien-21,17-carbolacton.

b) Zu einer Lösung von 4,3 g 15β, 16β-Methylen-3-pyrrolidino-19-nor-17α-pregna-3,5-dien-21,17-carbolacton in 43,6 ml Benzol und 87,2 ml Ethanol tropft man 4,3 ml Formalinlösung, rührt 1 Stunde bei Raumtemperatur nach und engt im Vakuum ein. Das erhaltene Rohprodukt wird an Kieselgel chromatographiert. Man erhält 1,9 g 6α-Hydroxymethyl-15β, 16β-methylen-3-oxo-19-nor-17α-pregn-4-en-21,17-carbolacton vom Schmelzpunkt 246,9 °C.

c) Zu einer Lösung von 2,06 g 6α-Hydroxymethyl-15β, 16β-methylen-3-oxo-19-nor-17α-pregn-4-en-21,17-carbolacton in 20 ml Pyridin gibt man 3,14 g p-Toluolsulfonsäure-chlorid, rührt 3 Stunden bei Raumtemperatur nach, versetzt mit 0,2 ml Wasser, rührt eine weitere Stunde und fällt die Reaktionslösung in Eiswasser. Der ausgefallene Niederschlag wird abfiltriert, mit Wasser gewaschen und getrocknet. Man erhält 2,39 g 15β, 16β-Methylen-3-oxo-6α-tosyloxymethyl-19-nor-17α-pregn-4-en-21,17-carbolacton.

d) Zu einer Lösung von 3,84 g Trimethylsulfoxoniumiodid in 75 ml Dimethylsulfoxid gibt man 566 mg 55 %iges Natriumhydrid und rührt 1 Stunde bei Raumtemperatur nach. Zu dieser Lösung tropft man 2,36 g 15β, 16β-Methylen-3-oxo-6α-tosyloxy-methyl-19-nor-17α-pregn-4-en-21,17-carbolacton in 2 ml Dimethylsulfoxid, rührt 30 Minuten nach und fällt in Eiswasser. Das erhaltene Rohprodukt wird an Kieselgel chromatographiert. Man erhält 985 mg 6,6-Ethylen-15β, 16β-methylen-3-oxo-19-nor-17α-pregn-4-en-21,17-carbolacton vom Schmelzpunkt 211-214 °C.

Die Ausgangsverbindung gemäß 6 a) wird wie folgt hergestellt :

Eine Lösung von 69,2 g 15α-Hydroxy-4-östren-3,17-dion in 408 ml Pyridin wird unter Eiskühlung mit 46,4 ml Benzoylchlorid versetzt und 1 Stunde bei Kühlung nachgerührt. Anschließend versetzt man mit 5,7 ml Wasser und rührt 3 Stunden bei Raumtemperatur nach und fällt in Eiswasser. Der ausgefallene Niederschlag wird abfiltriert, mit Wasser gewaschen und getrocknet. Man erhält 98,7 g 15α-Benzoyloxy-4-östren-3,17-dion.

Eine Lösung von 98,7 g 15α-Benzoyloxy-4-östren-3,17-dion in 4,5 l Dichlormethan wird bei 0 °C mit 266,7 ml Ethylenglycol, 177,8 ml o-Ameisensäuretriethylester und 889 mg p-Toluolsulfonsäure versetzt und 2,5 Stunden bei dieser Temperatur nachgerührt. Anschließend versetzt man mit etwas Pyridin und wäscht mehrmals mit Wasser. Nach dem Trocknen über Magnesiumsulfat engt man im Vakuum ein. Das erhaltene Rohprodukt wird an Kieselgel chromatographiert. Man erhält 69,1 g 15α-Benzoyloxy-3,3-ethylendioxy-5 bzw. 5(10)-östren-17-on.

Eine Lösung von 47,0 g Trimethylsulfoxoniumiodid in 1 l Dimethylsulfoxid wird mit 18,4 g 55 %igem Natriumhydrid versetzt und 1 Stunde unter Argon gerührt. Dazu tropft man eine Lösung von 69,1 g 15α-Benzoyloxy-3,3-ethylendioxy-5 bzw. 5(10)-östren-17-on und rührt 1 Stunde bei Raumtemperatur nach. Anschließend wird die Reaktionslösung in Eiswasser eingerührt, der ausgefallene Niederschlag abfiltriert und getrocknet. Das erhaltene Rohprodukt wird an Aluminiumoxid chromatographiert. Man erhält 41,5 g 3,3-Ethylendioxy-15β,16β-methylen-5 bzw. 5(10)-östren-17-on als Öl.

Eine Lösung von 41,5 g 3,3-Ethylendioxy-15β,16β-methylen-5 bzw. 5(10)-östren-17-on in 800 ml Tetrahydrofuran wird bei 0 °C unter Argon mit 148 g Kaliumethylat und anschließend mit einer Lösung von 57 ml Propargylalkohol in 57 ml Tetrahydrofuran versetzt und 2,5 Stunden bei Raumtemperatur nachgerührt. Anschließend säuert man mit Schwefelsäure an, verdünnt mit Wasser, extrahiert mit Essigester und wäscht mit Wasser neutral. Nach dem Trocknen und Einengen im Vakuum wird der Rückstand an Kieselgel crhomatographiert. Man erhält 38,0 g 17β-Hydroxy-17α-(3-hydroxy-1-propinyl)-15β,16β-methylen-4-östren-3-on vom Schmelzpunkt 161,3 °C.

Eine Lösung von 38,0 g 17β-Hydroxy-17α-(3-hydroxy-1-propinyl)-15β,16β-methylen-4-östren-3-on in 1 l Tetrahydrofuran wird mit 5,0 g Tristriphenylphosphinrhodium-I-chlorid hydriert und anschließend im Vakuum eingeengt. Man erhält 43,0 g 17β-Hydroxy-17α-(3-hydroxypropyl)-15β,16β-methylen-4-östren-3-on.

Eine Lösung von 43,0 g 17β-Hydroxy-17α-(3-hydroxypropyl)-15β,16β-methylen-4-östren-3-on in 1,4 l Dimethylformamid wird mit 160 g Pyridindichromat versetzt und über Nacht bei Raumtemperatur gerührt. Die Reaktionslösung wird anschließend in 7 l Essigester eingerührt, von den ausgefallenen Chromsalzen abgesaugt und das Filtrat mit Wasser gewaschen. Nach dem Eindampfen wird der Rückstand an Kieselgel chromatographiert. Man erhält 22,5 g 15β,16β-Methylen-19-nor-3-oxo-17α-pregn-4-en-21,17-carbolacton vom Schmelzpunkt 191,2 °C.

Beispiel für die Zusammensetzung eines oral zu applizierenden Kontrazeptivums in Form eines Dragées

Kern :

| | |
|---|---|
| 2,000 mg | 6,6-Ethylen-15β,16β-methylen-3-oxo-17α-pregn-4-en-21,17-carbolacton |
| 0,050 mg | Äthinylöstradiol |
| 31,100 mg | Lactose |
| 18,000 mg | Maisstärke |
| 2,100 mg | Poly-N-vinylpyrrolidon |
| 1,650 mg | Talkum |
| 0,100 mg | Magnesiumstearat |
| 55,000 mg | Gesamtgewicht, das mit üblicher Saccharosemischung (Hülle) auf ca. 90 mg ergänzt wird. |

Pharmakologische Beobachtungen

Die Antialdosteronwirkung wird im Testmodell von Hollmann (G. Hollmann et al., Tubuläre Wirkungen und renale Elimination von Spironolactonen, Naunyn-Schmiedebergs Arch. Exp. Path. Pharmak. 247 (1964), S. 419 ; P. Marx, Renale Wirkungen des d-Aldosterons und seines Antagonisten Spironolacton, Diss. Med. Fak. FU Berlin, 1966) festgestellt und gemessen.

Die Ergebnisse der gestagenen Wirkung werden im Clauberg-Test nach subcutaner Applikation der Wirkstoffe an kastrierten weiblichen Kaninchen erhalten. In den histologischen Schnitten wird die sekretorische Umwandlung des Endometriums bestimmt. Die Bestimmung erfolgt nach der McPhail-Skala (Beurteilungsgrade 1 - 4 ; 1 = keine Umwandlung ; 4 = vollständige Umwandlung).

Im Schwangerschaftserhaltungstest an Ratten wird nach subcutaner Verabreichung der Testsubstanzen vom 8. bis 21. Tag der Gravidität die Zahl der lebenden und toten Foeten ermittelt und die prozentuale Schwangerschaftserhaltung berechnet.

Wie aus der folgenden Tabelle hervorgeht, besitzen die erfindungsgemäßen Verbindungen 2 und 3 bei guter aldosteronantagonistischer Wirkung eine stärkere gestagene Potenz als die Vergleichssubstanz 1.

(Siehe Tabelle Seite 9 f.)

| Nr. | Substanz | Antialdosteron-Test p.o. (Spironolacton = 1) | Clauberg-Test s.c. Dosis /mg/ | Clauberg-Test s.c. McPhail-Wert | Schwangerschafts-erhaltungs-Test s.c. Dosis /mg/ | Schwangerschafts-erhaltungs-Test s.c. % |
|---|---|---|---|---|---|---|
| 1 | 6β,7β;15β,16β-Dimethylen-3-oxo-17α-pregn-4-en-21,17-carbolacton (als Vergleich) | 5 - 7 | 1<br>0,3<br>0,1 | 3,2<br>2,1<br>1,1 | 3<br>1<br>0,3<br>0,1 | 85<br>74<br>3<br>0 |
| 2 | 6,6-Ethylen-15β,16β-methylen-3-oxo-17α-pregn-4-en-21,17-carbolacton | 5 | 0,3<br>0,1<br>0,03 | 2,9<br>2,6<br>1,3 | 0,1<br>0,03<br>0,01 | 60<br>11<br>0 |
| 3 | 6,6-Ethylen-15β,16β-methylen-3-oxo-19-nor-17α-pregn-4-en-21,17-carbolacton | 1 | 0,3 | 2,8 | | |

**Patentansprüche** (für die Vertragsstaaten : BE, CH, DE, FR, GB, IT, LI, LU, NL, SE)

1. 6,6-Ethylen-15,16-methylen-3-oxo-17α-pregn-4-en-21,17-carbolactone der allgemeinen Formel I

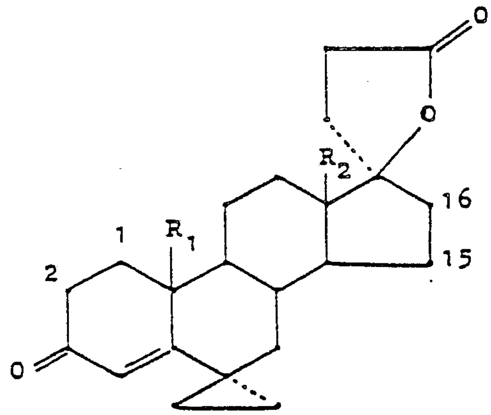

(I)

worin

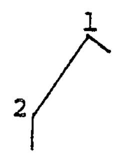

eine CC-Einfach- oder CC-Doppelbindung,
$R_1$ ein Wasserstoffatom oder eine Methylgruppe,
$R_2$ eine Methyl- oder Ethylgruppe und

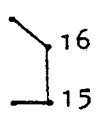 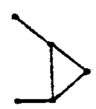 oder 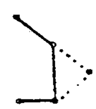

bedeuten.

2. 6,6-Ethylen-15β,16β-methylen-3-oxo-17α-pregn-4-en-21,17-carbolacton.
3. 6,6-Ethylen-15β,16β-methylen-3-oxo-17α-pregna-1,4-dien-21,17-carbolacton.
4. 6,6-Ethylen-15α,16α-methylen-3-oxo-17α-pregn-4-en-21,17-carbolacton.
5. 6,6-Ethylen-15α,16α-methylen-3-oxo-17α-pregna-1,4-dien-21,17-carbolacton.
6. 6,6-Ethylen-18-methyl-15β,16β-methylen-3-oxo-19-nor-17α-pregn-4-en-21,17-carbolacton.
7. 6,6-Ethylen-15β,16β-methylen-3-oxo-19-nor-17α-pregn-4-en-21,17-carbolacton.
8. Pharmazeutische Präparate, gekennzeichnet durch den Gehalt an Verbindungen gemäß Anspruch 1 bis 7.
9. Verfahren zur Herstellung von 6,6-Ethylen-15,16-methylen-3-oxo-17α-pregn-4-en-21,17-carbolactonen der allgemeinen Formel I

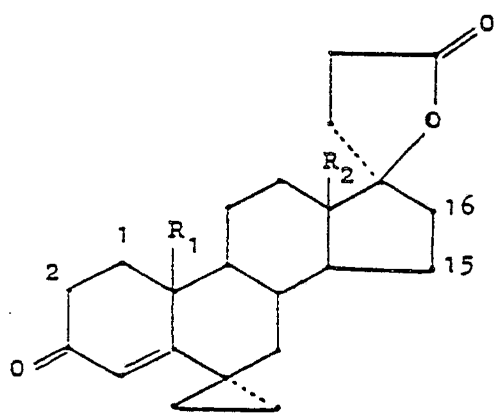

(I)

worin

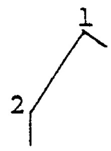

eine CC-Einfach- oder CC-Doppelbindung,

$R_1$ ein Wasserstoffatom oder eine Methylgruppe,

$R_2$ eine Methyl- oder Ethylgruppe und

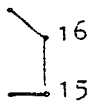

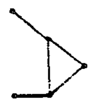

oder

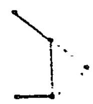

bedeuten, dadurch gekennzeichnet, daß man in 15,16-Methylen-3-oxo-17α-pregn-4-en-21,17-carbolactone der allgemeinen Formel II

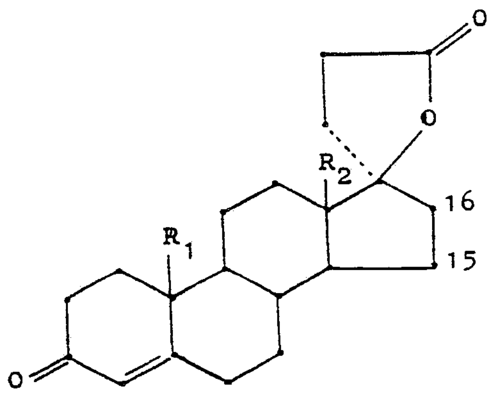

(II)

worin

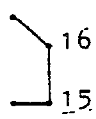

$R_1$ und $R_2$ die in Formel I angegebene Bedeutung haben, über die 3,5-Dien-3-Amine mit Formalin unter Rückbildung des 4-En-3-Oxo-Systems eine 6α-Hydroxymethylgruppe einführt, aus der 6α-Hydroxymethylverbindung die 6-Methylenverbindung bildet und diese zur 6,6-Ethylenverbindung methyleniert und gegebenenfalls die $\Delta^1$-Doppelbindung einführt.

**Patentanspruch** (für den Vertragsstaat AT)

Verfahren zur Herstellung von 6,6-Ethylen-15,16-methylen-3-oxo-17α-pregn-4-en-21,17-carbolactonen der allgemeinen Formel I

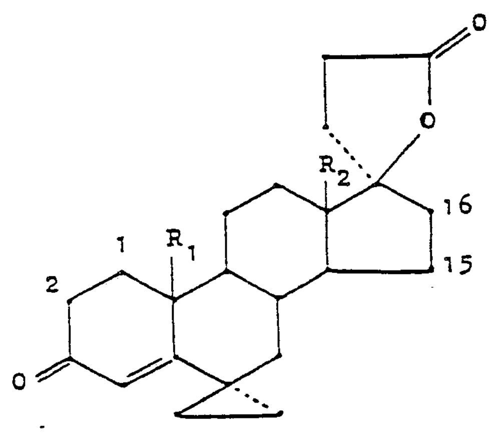

(I)

worin

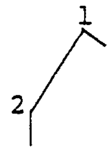

eine CC-Einfach- oder CC-Doppelbindung,
$R_1$ ein Wasserstoffatom oder eine Methylgruppe,
$R_2$ eine Methyl- oder Ethylgruppe und

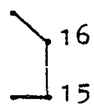 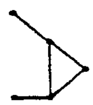 oder 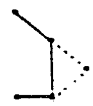

bedeuten, dadurch gekennzeichnet, daß man in 15,16-Methylen-3-oxo-17α-pregn-4-en-21,17-carbolactone der allgemeinen Formel II

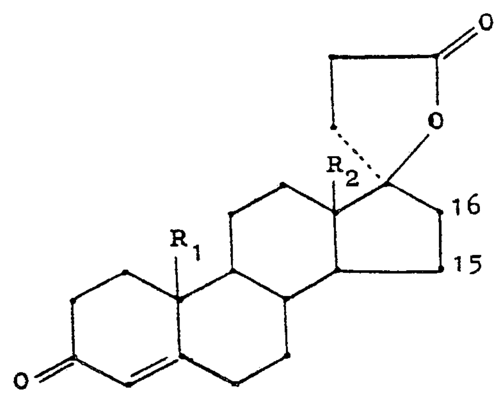

(II)

worin

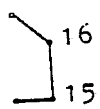

$R_1$ und $R_2$ die in Formel I angegebene Bedeutung haben, über die 3,5-Dien-3-Amine mit Formalin unter Rückbildung des 4-En-3-Oxo-Systems eine 6α-Hydroxymethylgruppe einführt, aus der 6α-Hydroxymethylverbindung die 6-Methylenverbindung bildet und diese zur 6,6-Ethylenverbindung methyleniert und gegebenenfalls die $\Delta^1$-Doppelbindung einführt.

**Claims** (for the Contracting States : BE, CH, DE, FR, GB IT, LI, LU, NL, SE)

1. 6,6-Ethylene-15,16-methylen-3-oxo-17α-pregn-4-ene-21,17-carbolactones of general formula I

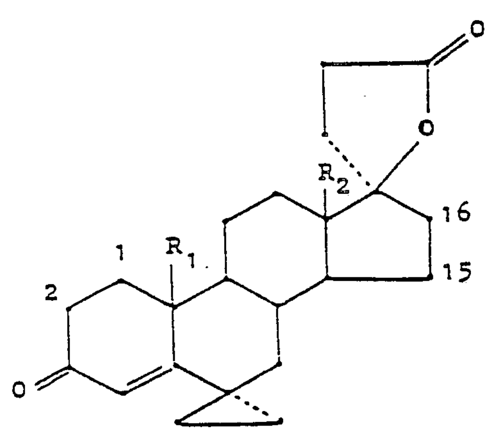

(I)

wherein

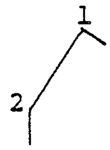

is a CC single or CC double bond,

$R_1$ is a hydrogen atom or a methyl group,

$R_2$ is a methyl or ethyl group, and

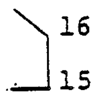 represents 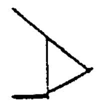 or 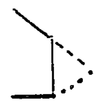

2. 6,6-Ethylene-15β,16β-methylene-3-oxo-17α-pregn-4-ene-21,17-carbolactone.
3. 6,6-Ethylene-15β,16β-methylene-3-oxo-17α-pregna-1,4-diene-21,17-carbolactone.
4. 6,6-Ethylene-15α,16α-methylene-3-oxo-17α-pregn-4-ene-21,17-carbolactone.
5. 6,6-Ethylene-15α,16α-methylene-3-oxo-17α-pregna-1,4-diene-21,17-carbolactone.
6. 6,6-Ethylene-18-methyl-15β,16β-methylene-3-oxo-19-nor-17α-pregn-4-ene-21,17-carbolactone.
7. 6,6-Ethylene-15β,16β-methylene-3-oxo-19-nor-17α-pregn-4-ene-21,17-carbolactone.
8. Pharmaceutical preparations, charaterised in that they contain compounds according to claims 1 to 7.
9. Process for the production of 6,6-ethylene-15,16-methylene-3-oxo-17α-pregn-4-ene-21,17-carbolactones of general formula I

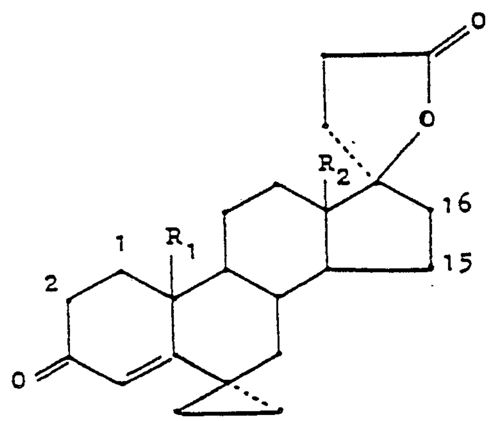

(I)

wherein

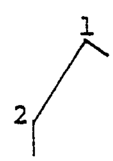

is a CC single or CC double bond,

$R_1$ is a hydrogen atom or a methyl group,

$R_2$ is a methyl or ethyl group, and

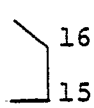 represents 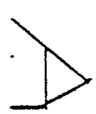 or 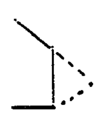

characterised in that a 6α-hydroxymethyl group is introduced into 15,16-methylene-3-oxo-17α-pregn-4-ene-21,17-carbolactones of general formula II

(See formula p. 14)

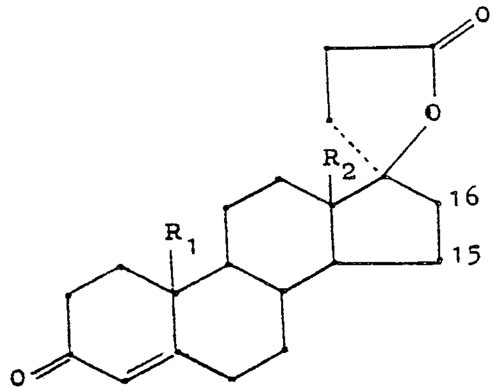

(II)

wherein

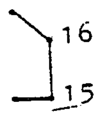

$R_1$ and $R_2$ have the meanings given in formula I, by way of the 3,5-diene-3-amines with the use of formalin, with reconstitution of the 4-ene-3-oxo system ; the 6-methylene compound is formed from the 6α-hydroxymethyl compound ; and the 6-methylene compound is methylenated to form the 6,6-ethylene compound ; and optionally the $\Delta^1$ double bond is introduced.

**Claim** (for the Contracting State AT)

Process for the production of 6,6-ethylene-15,16-methylene-3-oxo-17α-pregn-4-ene-21,17-carbolactones of general formula I

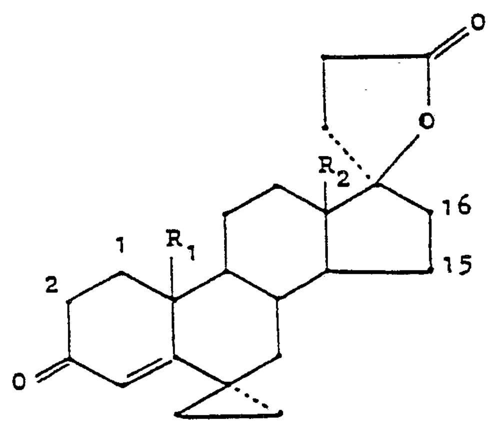

(I)

wherein

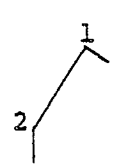

is a CC single or CC double bond,
$R_1$ is a hydrogen atom or a methyl group,
$R_2$ is a methyl or ethyl group, and

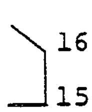 represents 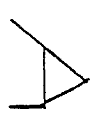 or 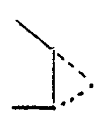

characterised in that a 6α-hydroxymethyl group is introduced into 15,16-methylene-3-oxo-17α-pregn-4-ene-21,17-carbolactones of general formula II

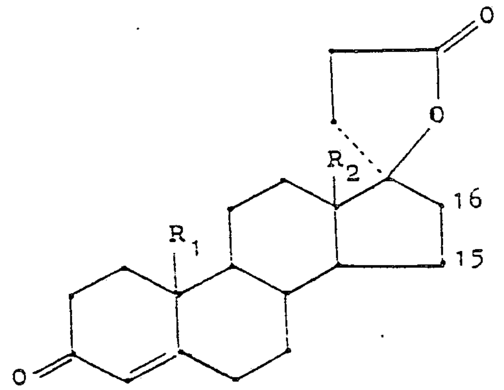

(II)

wherein

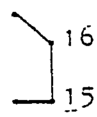

$R_1$ and $R_2$ have the meanings given in formula I, by way of the 3,5-diene-3-amines with the use of formalin, with reconstitution of the 4-ene-3-oxo system ; the 6-methylene compound is formed from the 6α-hydroxymethyl compound ; and the 6-methylene compound is methylenated to form the 6,6-ethylene compound ; and optionally the $\Delta^1$ double bond is introduced.

**Revendications** (pour les Etats contractants : BE, CH, DE, FR, GB, IT, LU, LI, NL, SE)

1. Ethylène-6,6 méthylène-15,16 oxo-3 17α-prégnène-4 carbolactones-21,17 qui répondent à la formule générale I :

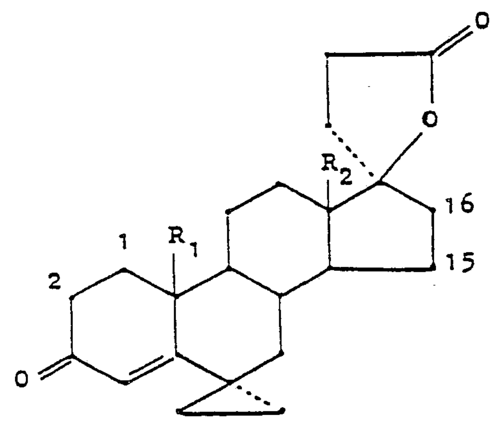

(I)

dans laquelle

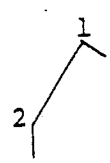

représente une liaison carbone-carbone simple ou double,

$R_1$ représente un atome d'hydrogène ou un radical méthyle,

$R_2$ représente un radical méthyle ou éthyle et représente

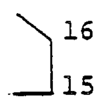 représente 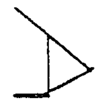 ou 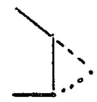

2. Ethylène-6,6 méthylène-15β,16β oxo-3 17α-prégnène-4 carbolactone-21,17.

3. Ethylène-6,6 méthylène-15β,16β oxo-3 17α-prégnadière-1,4 carbolactone-21,17.

4. Ethylène-6,6 méthylène-15α,16α oxo-3 17α-prégnène-4 carbolactone-21,17.

5. Ethylène-6,6 méthylène-15α,16α oxo-3 17α-prégnadiène-1,4 carbolactone-21,17.

6. Ethylène-6,6 méthyl-18 méthylène-15β,16β oxo-3 nor-19 17α-prégnène-4 carbolactone-21,17.

7. Ethylène-6,6 méthylène-15β,16β oxo-3 nor-19 17α-prégnène-4 carbolactone-21,17. .

8. Compositions pharmaceutiques caractérisées en ce qu'elles contiennent des composés selon l'une quelconque des revendications 1 à 7.

9. Procédé de préparation d'éthylène-6,6 méthylène-15,16 oxo-3 17α-prégnène-4 carbolactones-21,17 répondant à la formule générale I :

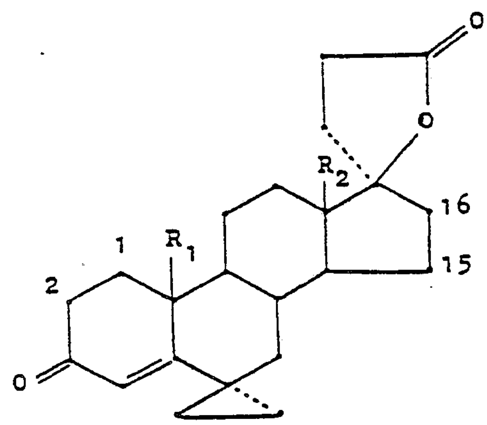

(I)

dans laquelle

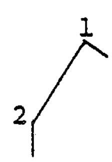

représente une liaison carbone-carbone simple ou double,

$R_1$ représente un atome d'hydrogène ou un radical méthyle,

$R_2$ représente un radical méthyle ou éthyle et représente

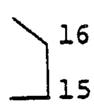 représente 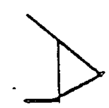 ou 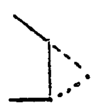

procédé caractérisé en ce que, dans les méthylène-15,16 oxo-3 17α-prégnène-4 carbolactones-21,17 répondant à la formule générale II :

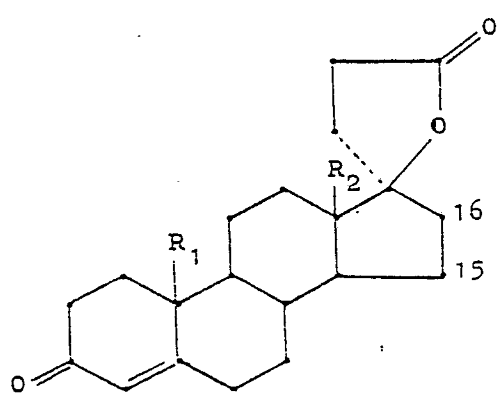

(II)

dans laquelle

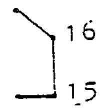

$R_1$ et $R_2$ ont les significations données à propos de la formule I, on introduit, en passant par les diène-3,5 amines-3, au moyen du formaldéhyde, avec régénération du système ène-4 oxo-3, un radical hydroxyméthyl-6α, on forme, à partir du composé hydroxyméthyl-6α, le composé méthylène-6, on méthylène celui-ci de manière à le convertir en le composé éthylène-6,6 et, éventuellement, on introduit la double liaison $\Delta^1$.

**Revendication** (pour l'Etat contractant AT)

Procédé de préparation d'éthylène-6,6 méthylène-15,16 oxo-3 17α-prégnène-4 carbolactones-21,17 répondant à la formule générale I :

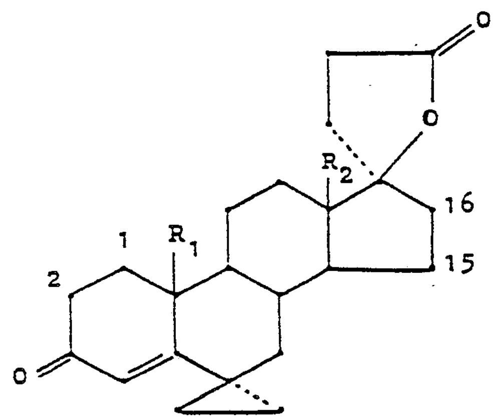

(I)

dans laquelle

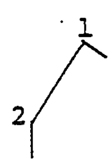

représente une liaison carbone-carbone simple ou double,

$R_1$ représente un atome d'hydrogène ou un radical méthyle,

$R_2$ représente un radical méthyle ou éthyle et

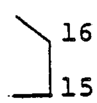 représente 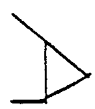 ou 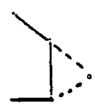

procédé caractérisé en ce que, dans des méthylène-15,16 oxo-3 17α-prégnène-4 carbolactones-21,17 répondant à la formule générale II :

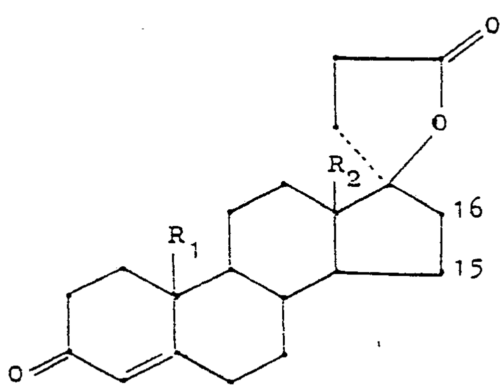

(II)

dans laquelle

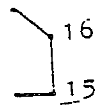

$R_1$ et $R_2$ ont les significations données à propos de la formule I, on introduit, en passant par les diène-3,5 amines-3, au moyen du formaldéhyde, avec régénération du système ène-4 oxo-3, un radical hydroxyméthyl-6α, on forme, à partir du composé hydroxyméthyl-6α, le composé méthylène-6, on méthylène celui-ci de manière à le convertir en le composé éthylène-6,6 et, éventuellement, on introduit la double liaison $\Delta^1$.